# EUROPEAN PATENT APPLICATION

(11) **EP 1 623 724 A1**
(43) Date of publication of application: **08.02.2006**
(21) Application number: 05100593.2
(22) Date of filing: 28.01.2005
(51) Int. Cl.: A61L 2/10, C02F 1/32

(54) **Autoclave with UV tank irradiation and associated tank lid**

(30) Priority: 28.07.2004 IT MI20041535
(71) Applicant: W & H Sterilization S.r.l., 24066 Pedrengo, Bergamo (IT)
(72) Inventor: Ongaro, Daniele, 24020, Villa di Serio (Bergamo) (IT); Cattaneo, Giulia, 24052, Azzano S. Paolo (Bergamo) (IT); Samanni, Emanuele, 24126, Bergamo (IT); Ghezzi, Fabio, 20095, Cusano Milanino (Milano) (IT)
(74) Representative: Faggioni, Carlo Maria

(57) **Abstract**

A ambulatory autoclave for the sterilisation of medical instruments is disclosed, comprising at least one sterilisation chamber, means to empty said chamber, and one or more water tanks, wherein it is provided a source of UV rays configured so as to radiate said one or more tanks.

## Description

The present invention relates to an autoclave for the sterilisation of medical tools, in particular for use in dentistry, equipped with a low-bacterial-charge environment.

So-called fractionated vacuum autoclaves are widely known in the field of ambulatory sterilisation equipments for medical instruments. In this type of equipment, sterilisation of small medical instruments and tools (for example contra-angle handpieces used in dentistry) is accomplished through a series of vacuum and exposure to steam cycles. The instruments are introduced in a sterilisation chamber, from which air is sucked (which would otherwise hinder sterilisation, since it would act as an insulating agent) by a vacuum pump and in which a series of high-temperature steam input and output cycles are then run.

The gaseous contents of the sterilisation chamber (air or steam mixed with air) are emptied by means of a pump and discharged into a suitable storage tank - usually located inside the device casing - where the condensed steam provisionally collects and accumulates in the form of water before being definitively disposed of in a sanitary sink at the ambulatory clinic.

The first step of vacuum creation in the sterilisation chamber is the most critical one from a sanitary point of view: as a matter of fact, in this procedure - since the sterilising effect of heat has not yet begun - the humid air sucked by the pump contains a heavy charge of micro-organisms which reside on the instruments loaded into the autoclave. Since the storage tank is not sealed, of course, the air coming thereto, pushed by the pump, inevitably ends up invading the entire work environment, i.e. the ambulatory clinic rooms.

As can be guessed, this fact brings sanitary problems of environmental pollution, in an environment, that of dentistry, which must have a low bacterial charge to allow correct storage of the sterilised material.

This disadvantage is sharpened by the fact that the collection tank is not emptied frequently by medical staff and hence becomes itself an ideal breeding ground for the development of micro-organisms, turning into a significant source of bacteria which contaminate the environment outside the autoclave.

Among other things, since often - for ease of installation and construction inexpensiveness - the storage tank is mounted in the autoclave adjacent to the clean water tank which serves to supply the steam generator with water, the bacterial charge can easily transfer from one tank to the other, further compromising the sanitary features of the autoclave. As a matter of fact, the presence of bacteria in the supply water of the steam generator, makes it more difficult from the start to reach the SAL (Sterility Assurance Level), required to ensure sterility.

There is more, however. The Applicant could ascertain that in the plants where a demineralising cartridge is employed, installed in the supply pipe of the freshwater tank (to avoid buying demineralised water in large bottles), there are further bacterial charge problems. As a matter of fact, the usually resin-based filtering cartridge represents a relevant receptacle and breeding ground for bacteria, which therefore arrive in great numbers at the supply tank even before the autoclave is switched on.

According to prior art, a first approach has already been made to solve this problem, providing separate equipment for destruction of the bacterial charge, wherein the air sucked from the sterilisation chamber is suitably treated, before being released into the outer environment. In this piece of equipment (refer for example to the device Aria^{TM} marketed by Tecno-Gaz) the sucked air is subjected to a hard treatment in order to immediately destroy the charge of micro-organisms.

However, having stated the above, this solution is not satisfying because, as well as being expensive, space-consuming and awkward to use, it does not remove the bacterial charge present in the still water of the collection tank and in the water of the supply tank.

Moreover, this piece of additional equipment must be suitably interfaced with the autoclave, which implies modification operations which cannot always be carried out by medical staff.

Besides, the storage of still water, even having a low bacterial charge, in the autoclave freshwater tank, inevitably causes a considerable increase in the bacterial charge, since the bacteria breed rapidly. It must be kept in mind that the destruction of a large quantity of bacteria causes release of endotoxins in the steam. Endotoxins are heat resistant and may survive to the sterilisation process: they therefore collect on dentistry equipment, which should be avoided, since they, as it is widely known, may cause tissue inflammation. It is therefore very important to avoid bacterial breeding in the freshwater tank, so as to keep down the number of bacteria and to thereby lessen the quantity of endotoxin in the steam.

It is an object of the present invention to provide an autoclave which is arranged so as to always keep the bacterial charge in the work environment thereof very low, without that causing significant extra costs, complications in the use of the device or significant bulk increases.

Such an object is achieved by means of an autoclave as described in its essential features in the attached claims.

Further details and advantages of the autoclave according to the invention will appear more evident from the following detailed description, taken together with the accompanying drawings, wherein:

fig. 1 is a top perspective exploded view of a double tank according to a first embodiment of the invention;

fig. 2 is a view as in fig. 1 of the assembled double tank;

fig. 3 is a bottom perspective view of the tank of fig. 2;

fig. 4 is a top perspective view of a second embodiment of the invention;

figg. 5A-5C are axonometric views of the tank of fig. 4;

fig. 6 is a top perspective view of a third embodiment of the invention;

fig. 7 is a perspective view of the tank of fig. 6, taken from a closer position and from the mounting side of the lamp; and

fig. 8 is a section view of the tank of fig. 6 taken longitudinally along the lamp.

An ambulatory autoclave comprises, in a manner known per se, a series of devices and apparatuses apt to sterilise by heat a plurality of objects for medical use.

As mentioned before, a particularly sensitive element - from a sanitary point of view - among the various devices is the collection tank of the gaseous and condensed contents coming from the sterilisation chamber.

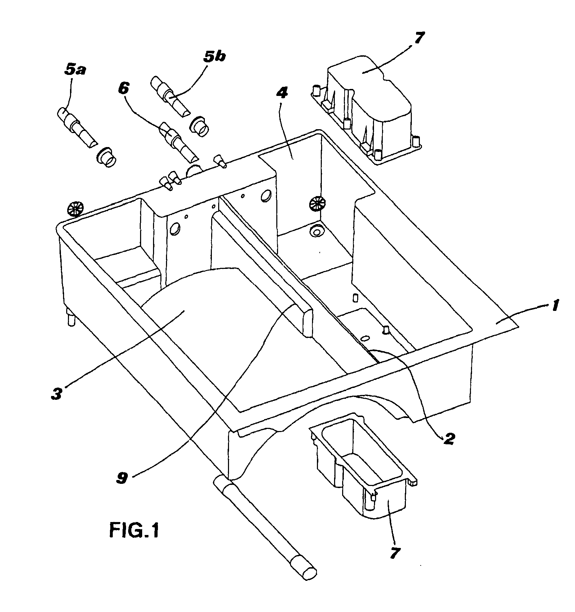

In fig. 1 a double exemplary tank is shown. In a container 1, having a generally rectangular perimeter, are defined two adjacent reservoirs, separated by a partition 2: a first collection reservoir 3 (intended to supply the steam generator) and a freshwater containment reservoir 4.

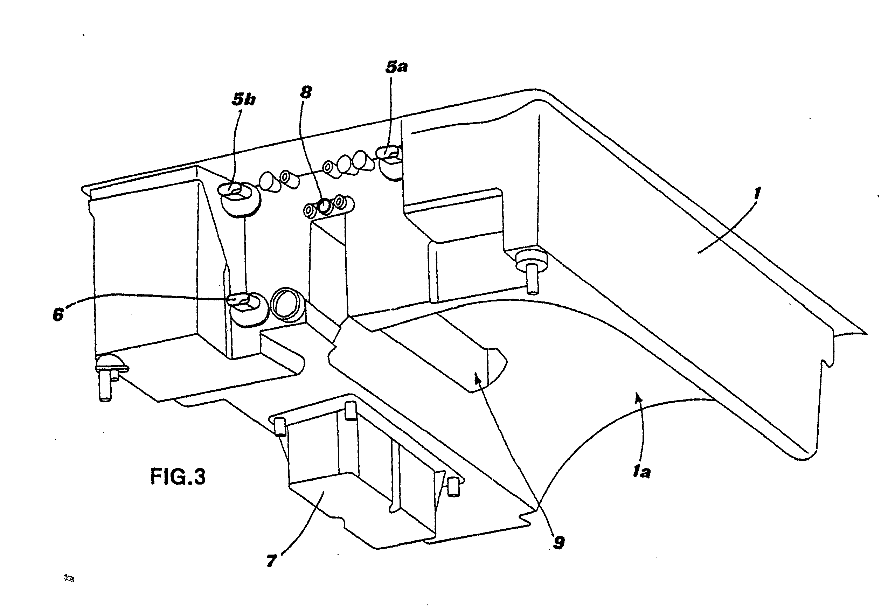

Container 1 is preferably shaped so as to be able to be housed above the autoclave sterilisation chamber: for such purpose, it has a deep saddle-shaped recess 1a, as is well represented in fig. 3.

On the back wall of container 1, in the proximity of the upper edge, are inserted two sensors 5a and 5b apt to detect the water overflow level, in reservoir 3 and 4, respectively. In the same wall of container 1, in the lower part of the freshwater reservoir 4, another detection probe 6 of the lower level is provided.

In the freshwater reservoir 4 a condensation discharge device 7 is further housed, also called "water trap", mounted on the two opposite sides of the bottom wall of container 1 and described in detail in EP 1.273.310 in the name of the same Applicant.

According to a first embodiment of the invention, furthermore, across the two reservoirs 3 and 4, inside partition 2, a UV-emitting lamp 8 is provided.

For such purpose, partition 2 advantageously has a hollow enlargement which defines a housing chamber 9 for lamp 8. The walls of chamber 9 in partition 2 are transparent to UV rays and hence allow UV radiation to light up reservoirs 3 and 4 from a level higher than the free surface of the water therein contained if the tank is not full. If the tank is full of water up to the maximum level, the lamp lights up not only the free surface of the water, but also the water itself remaining at least partly immersed therein.

In the lower part of container 1, the housing chamber 9 of lamp 8 is open on the back and bottom sides as can be seen in fig. 3: in this way, as well as easing fitting and unfitting of the UV lamp 8, a shape of container 1 is determined so that it can be easily removed from its manufacturing mould.

The UV lamp 8 can easily be mounted and unmounted from container 1 for ordinary maintenance and/or replacement operations. It is either always kept switched on or it is provided that, at the end of each sterilisation cycle, it stays on for a pre-established length of time, for example at least 36 hours.

For such purpose, according to a preferred embodiment, the UV lamp 8 is powered by a controller completely independent from the one driving the autoclave operation. This allows to render the bacterial charge control activity completely independent from the autoclave operation, ensuring best results regardless of the operation of the autoclave. In particular, it is possible to keep the UV lamp switched on even for 36 hours after the last sterilisation cycle, although the autoclave is switched off completely.

Advantageously, thanks to the UV lamp control independence, it is possible to provide a work cycle which switches on the UV source even before loading water into the two reservoirs 3 and 4: this option substantially allows to sanitise the reservoirs and to arrange them in the best conditions for the successive maintenance of a low bacterial charge.

Preferably, a microswitch is further provided which interrupts operation of the UV lamp in case a covering lid of container 1 or of another covering means is lifted allowing access to the UV lamp from the outside. This prevents the electromagnetic rays coming from the UV lamp to end up radiating other unscheduled objects or people, with the risk of personal injury.

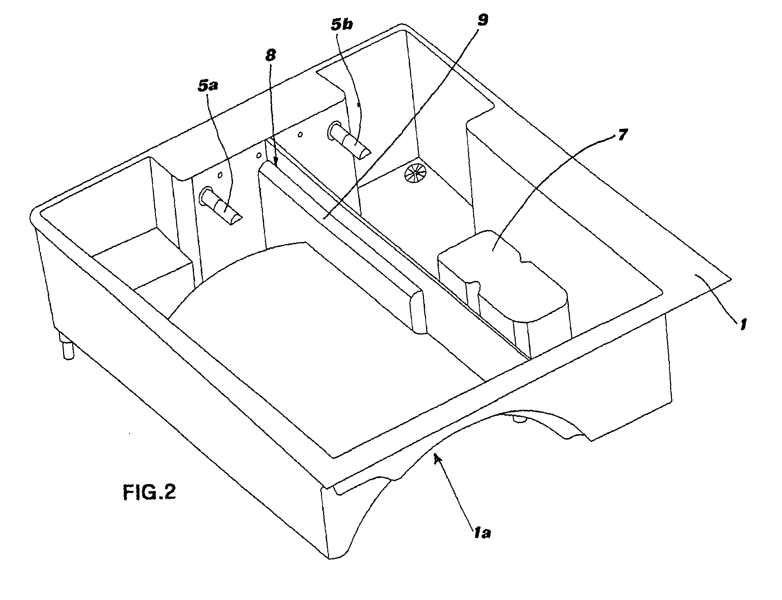

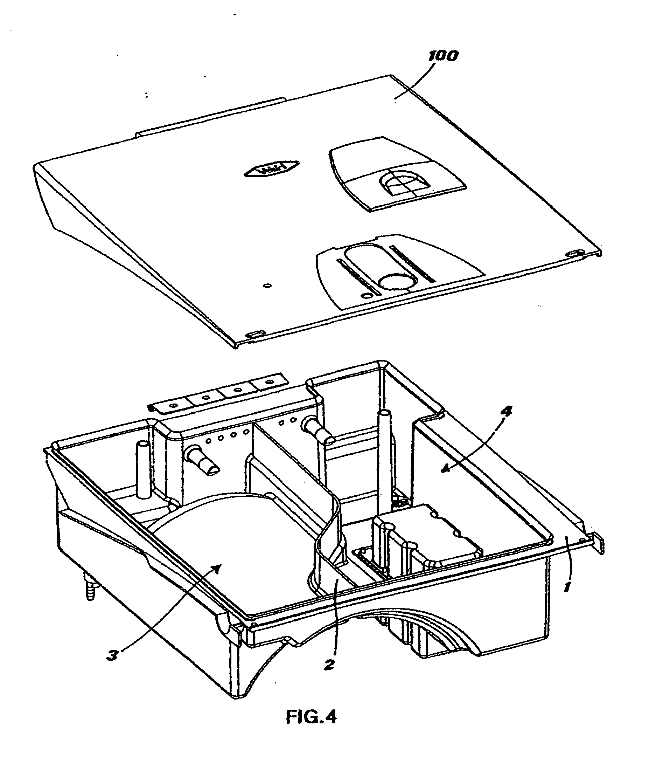

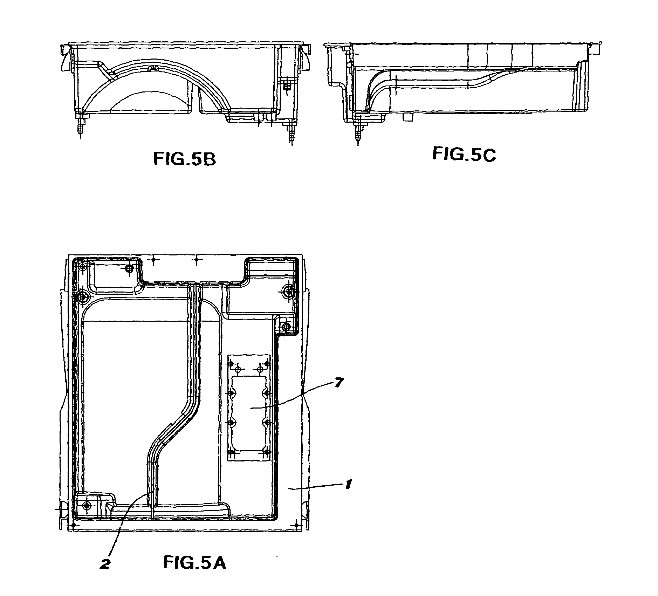

In figs. 4 and 5 a second embodiment of the invention is shown, wherein the elements corresponding to figs. 1-3 have the same reference numbers.

In this case partition 2 has no room for the UV lamp. Viceversa, the latter (not visible in fig. 4) is housed on the lower side of a tank covering lid 100. The UV lamp is preferably arranged so as to extend on both reservoirs 3 and 4 and hence radiate the water free surface (or also the water itself when it remains partly immersed with a full reservoir) therein contained.

At the interconnection between the lid 100 and the twin tank, as also seen above, a safety microswitch (not shown) is suitably provided, apt to disable functioning of the lamp 8 when the lid is lifted from the tank.

Advantageously, such a lid, provided with an autonomous UV source, possibly equipped with a respective controller, can easily be applied to compatible twin tanks and can hence represent a valid additional equipment to be provided on already existing compatible autoclaves.

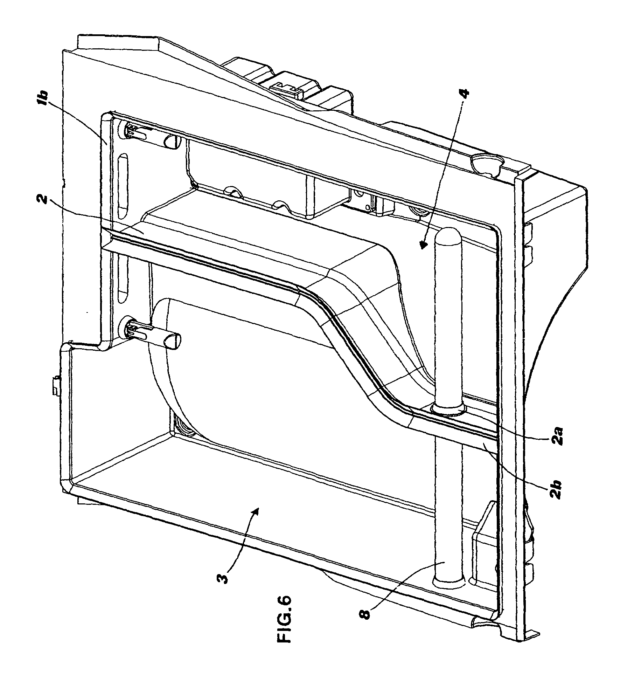

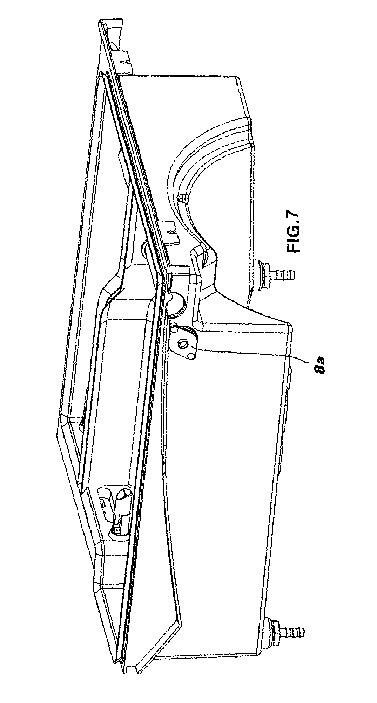

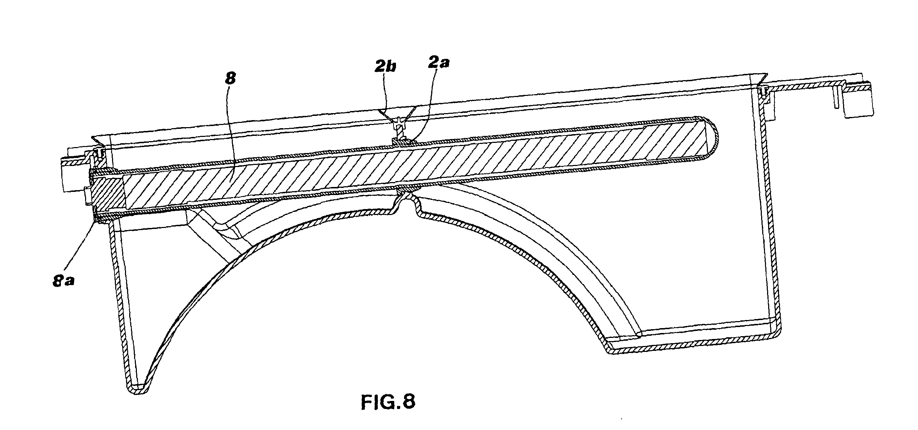

In figs. 6-9 a third embodiment of the invention is shown, wherein the elements corresponding to figs. 1-3 have the same reference numbers.

Here the UV lamp 8 is lying across the two freshwater and collection reservoirs, transversally mounted through the partition so as to effectively radiate both reservoirs.

Lamp 8 is mounted cantilevering from the perimeter wall of reservoirs 3, as is well visible in figs. 6 and 9, through a suitable connector 8a through which it can be connected to power lines (not shown) situated externally of reservoir 1. Moreover, the lamp crosses partition 2 at a respective hole equipped with a seal 2a which, as well as supporting lamp 8, reduces the possibilities of cross-contamination between reservoirs.

Advantageously, the upper edge of the entire partition 2 is equipped with a lip seal 2b intended to provide a seal against the lower surface of the above lid (not shown), thereby ensuring a similar function to seal 2a.

Preferably, a further lip seal 1b is provided along the entire perimeter of reservoirs 3 and 4.

The solution offered by the present invention perfectly achieves the objects set forth in the preamble. As a matter of fact, having available a UV lamp which lights up directly the collection tank collecting the gaseous and condensed contents emptied from the sterilisation chamber, the bacterial charge which arrives there is rapidly diminished, a successive breeding of micro-organisms is avoided, and hence a hygienic quality is obtained both of the collection reservoir and of the entire environment enclosed in the autoclave casing.

The fact then that the same lamp lights up the freshwater supply reservoir also, makes the system extremely effective in terms of the net benefit/investment ratio.

Again, in the arrangement illustrated in figs. 4-5, the device of the invention can be universally applied to a series of pre-existing twin tanks, thereby making itself particularly useful even in existing autoclaves if compatible with such a use.

It is understood, however, that protection of the invention described above is not limited to the particular arrangements illustrated, but extends to any other construction variant which exploits the same teachings and which falls within the scope of the claims as hereunder defined.

In particular, reference is made to the configuration of the collection reservoir which must not necessarily be integral with the freshwater tank.

It must be appreciated that, in the context of this description, by the term "ambulatory" autoclave, it is meant autoclaves of a size ranging from 1 to 50 litres, rather than strictly an equipment intended exclusively to equip a clinic.

## Claims

1. An ambulatory autoclave for the sterilisation of medical tools of the type comprising at least one sterilisation chamber, emptying means of said chamber, and one or more tanks for water, **characterised in that** it further comprises a source of UV rays arranged so as to radiate at least towards said one or more tanks.

2. The ambulatory autoclave as in claim 1), wherein at least one of said one or more tanks of water is a collection tank (3) wherein said emptying means deliver the gaseous and condensed contents from the sterilisation chamber, and said source of UV rays is arranged so as to radiate at least towards said collection tank.

3. The ambulatory autoclave as in claim 1) or 2), wherein at least one of said one or more tanks of water is a fresh-water tank (4) for supplying a steam generator, and wherein said source of UV rays radiates at least towards said fresh-water tank.

4. The ambulatory autoclave as in claim 2) and 3), comprising a collection tank (3) and a fresh-water tank (4), adjacent to each other.

5. The ambulatory autoclave as in claim 4), wherein said tanks (3, 4) are integrally made and are arranged above and across said sterilisation chamber.

6. The ambulatory autoclave as in 4) or 5), wherein said source of UV rays is mounted longitudinally or transversally onto a partition wall (2) of said tanks (3, 4) to radiate towards both, and wherein there are further provided switch means apt to automatically interrupt operation of said UV source upon opening of a lid of said tanks.

7. The ambulatory autoclave as in claim 6), wherein said UV source is a lamp (8) inserted in a housing chamber (9) obtained on said partition wall (2), the chamber walls being transparent to UV rays and the lamp being insertable into said chamber (9) from outside the tanks (3, 4).

8. The ambulatory autoclave as in claim 6), wherein said UV source is an elongated lamp (8) mounted cantilevered on a perimeter wall of one of said tanks and crossing said partition wall (2).

9. The ambulatory autoclave as in any one of the previous claims, wherein said source of UV rays is integral with a lid (100) intended to close from above said tanks (3, 4).

10. The ambulatory autoclave as in any one of the previous claims, wherein said source of UV rays is powered through a controller separate from the autoclave controller, so that it may operate even when the autoclave is off.

11. Closing lid for a twin tank for an autoclave as in claim 9), **characterised in that** it comprises a source of UV rays, arranged on the bottom side thereof, and a safety switch apt to interrupt operation of said UV source when the lid is raised from the respective tank.

12. Lid as in claim 11) and 10), wherein said controller of the UV ray source is independent and integral with the lid and may be interfaced with the autoclave basic power supply.
